Europäisches Patentamt

European Patent Office

Office européen des brevets

(11) Veröffentlichungsnummer: **0 267 384**
**A1**

(12) **EUROPÄISCHE PATENTANMELDUNG**

(21) Anmeldenummer: 87112973.0

(22) Anmeldetag: 04.09.87

(51) Int. Cl.⁴: **C07C 69/28** , C07C 67/58

(30) Priorität: 08.11.86 DE 3638167

(43) Veröffentlichungstag der Anmeldung:
18.05.88 Patentblatt 88/20

(84) Benannte Vertragsstaaten:
BE CH DE ES FR GB IT LI NL

(71) Anmelder: HÜLS AKTIENGESELLSCHAFT
Patentabteilung / PB 15 - Postfach 13 20
D-4370 Marl 1(DE)

(72) Erfinder: Engel, Klaus, Dr.
Lange Strasse 28
D-4100 Duisburg 14(DE)
Erfinder: Schüller, Hans-Peter
Alte Brüderstrasse 16
D-4370 Marl(DE)

(54) Verfahren zur Herstellung von Fettsäurepolyoxyalkylendiolmonoestern.

(57) Die Erfindung betrifft ein Verfahren zur Herstellung von reinen Fettsäurepolyoxyalkylendiolmonoestern. Dabei werden zunächst Fettsäuren oder Fettsäurederivate mit Polyoxyalkylendiolen umgesetzt. Aus den Reaktionsgemischen werden die Monoester bei erhöhter Temperatur mit Paraffin extrahiert. Beim Abkühlen der Paraffinextrakte scheiden sich die Monoester ab und können so auf einfache Weise gewonnen werden.

EP 0 267 384 A1

## Verfahren zur Herstellung von Fettsäurepolyoxyalkylendiolmonoestern

Die Erfindung betrifft ein Verfahren zur Herstellung von Fettsäurepolyoxyalkylendiolmonoestern durch Umsetzung von Fettsäuren oder Fettsäurederivaten mit überschüssigen Polyoxyalkylendiolen und extraktiver Abtrennung der reinen Monoester aus den Reaktionsgemischen.

Fettsäurepolyoxyalkylendiolmonoester sind nichtionische Tenside, die neben ihrer Wasch-und Reinigungswirkung insbesondere aufgrund ihrer geringen Toxizität sowie ihrer milden hautglättenden Wirkung vorzugsweise als Emulgatoren in kosmetischen Formulierungen eingesetzt werden. Sie dienen auch als milde Tenside für Shampoo-Zubereitungen, insbesondere für Baby-Shampoos.

Es ist bekannt, daß Fettsäurepolyoxyalkylendiolmonoester durch Direktoxethylierung von Fettsäuren oder durch Umsetzung von Polyoxyalkylendiolen mit Fettsäuren, Fettsäureanhydriden, Fettsäurechloriden oder Fettsäureestern in Gegenwart von sauren oder basischen Katalysatoren zugänglich sind. Neben den gewünschten Monoestern fallen dabei im allgemeinen auch Diester sowie unveresterte Polyoxyalkylendiole in erheblichem Umfange an. Beide Nebenprodukte beeinträchtigen die anwendungstechnische Qualität des gewünschten Hauptproduktes in starkem Maße.

Bei der Direktoxethylierung von Fettsäuren entstehen neben den Polyoxyethylendiolmonoestern in erheblichem Maße auch Polyoxyethylendioldiester. Eine Trennung dieser Produkte ist sehr aufwendig.

Nach DE-AS 19 01 535 werden Carbonsäuren und Ethylenoxid im Molverhältnis 1 : 1 eingesetzt und mit Hilfe eines Aminoxids als Katalysator zu Carbonsäurenethylenglykolmonoestern umgesetzt. Dieses Verfahren ist jedoch nicht auf Ester mit einem Polyoxyalkylendiol als Alkoholkomponente übertragbar.

Des weiteren wird in der DE-AS 11 75 220 vorgeschlagen, Fettsäuren mit überschüssigem Polyoxyalkylendiol umzusetzen und anschließend den Monoester durch Extraktion des überschüssigen Polyoxyalkylendiols mit einer wäßrigen Salzlösung abzutrennen und das Polyoxyalkylendiol in einer weiteren Extraktionsstufe mit Hilfe von Chloroform, Methylenchlorid oder Tetrahydrofuran aus der wäßrigen Salzlösung zu regenerieren.

Die durch diese verhältnismäßig aufwendige Extraktionstechnik gewonnenen Produkte enthalten verfahrensbedingt noch beträchtliche Anteile Wasser und gelöstes Salz. Bei hydrophilen Monoestern sind die Produktverluste durch die Extraktion erheblich. Darüber hinaus ist der Einsatz von chlorierten Kohlenwasserstoffen zur Regenerierung des Polyoxyalkylendiols für die Produktqualität des erwünschten Monoesters unvorteilhaft, besonders wenn das Produkt im Lebensmittel-oder Kosmetikbereich eingesetzt werden soll.

Man kann schließlich auch den Umweg über Schutzgruppen gehen. So werden beispielsweise über Borsäurezwischenprodukte reine Monoester erhalten (L. Hartman, J. Chem. Soc., London, 1957, S. 1918). Derartige Verfahren sind aufwendig und allgemein von keinem wirtschaftlichen Interesse.

Aufgabe der Erfindung ist es, ein verbessertes und technisch einfaches Verfahen zur Herstellung von Fettsäurepolyoxyalkylendiolmonoestern bereitzustellen. Die Produkte sollen nur geringe Mengen an Diestern und keine weiteren, die Produktqualität mindernde Nebenprodukte wie Wasser oder anorganische Salze und keine physiologisch bedenklichen Lösungsmittel enthalten.

Die Aufgabe wird erfindungsgemäß dadurch gelöst, daß man die Rohprodukte nach der Umsetzung von Fettsäuren oder Fettsäurederivaten mit überschüssigen Polyoxyalkylendiolen bei einer erhöhten Temperatur mit Paraffin extrahiert, die Paraffinphase anschließend abkühlt und die sich abtrennende Fettsäurepolyoxyalkylendiolmonoester-Phase separiert.

Für die Herstellung der Fettsäurepolyoxyalkylendiolmonoester können gesättigte oder ungesättigte Fettsäuren mit 5 bis 24 C-Atomen oder deren Mischungen verwendet werden. Geeignet sind beispielsweise Capronsäure, Caprylsäure, Caprinsäure, Laurinsäure, Myristinsäure, Palmitinsäure, Stearinsäure, Arachinsäure, Behensäure, Lignocerinsäure, Palmitoleinsäure, Ölsäure, Elaidinsäure, Linolsäure, Ricinolsäure, oder deren natürlichen Triglyceriden entsprechende Mischungen, wie z. B. Rübölfettsäure, Tallölfettsäure, Kokosfettsäure, Sojaölfettsäure, Ricinusölfettsäure, Palmkernölfettsäure, Palmölfettsäure, Erdnußölfettsäure, Baumwollsaatölfettsäurre, Sonnenblumenölfettsäure, Leinölfettsäure, Talgfettsäure oder Fischölfettsäurre.

Kokosfettsäure ist dabei ein Gemisch aus im wesentlichen Caprylsäure, Caprinsäure, Laurinsäure, Myristinsäure, Palmitinsäure und Ölsäure.

Die Polyoxyalkylendiole können durch Polymerisation von cyclischen Ethern mit 2 bis 4 C-Atomen hergestellt werden. Vorzugsweise verwendet man Polyoxyethylendiole mit mittleren Molmassen von 150 bis 2 000. Besonders bevorzugt sind Produkte mit mittleren Molmassen von 400 bis 1 500. Bei mittleren Molmassen < 150 wird die Wasserlöslichkeit und bei mittleren Molmassen > 2 000 die Fettlöslichkeit der Monoester zu gering.

Erfolgt eine Veresterung der Fettsäuren mit den Polyoxyalkylendiolen, so kann diese Reaktion mit Hilfe von alkalischen oder sauren Katalysatoren, wie beispielsweise Titantetraisopropylat, p-Toluolsulfonsäure oder anorganischen Mineralsäuren, durchgeführt werden. Üblicherweise erfolgt die Veresterung bei 120 bis 160 °C, wobei auch andere Umsetzungstemperaturen gewählt werden können. Durch Anlegen von Vakuum kann dabei die Destillation von Wasser beschleunigt werden.

Das Molverhältnis von Fettsäure zu Polyoxyalkylendiol beträgt 1 : 4 bis 1 : 10. Dabei werden Polyoxyalkylendiole mit hoher Molmasse in eher geringerem molaren Überschuß und Polyoxyalkylendiole mit niedriger Molmasse in eher höherem molaren Überschuß eingesetzt.

Üblicherweise enthalten die so hergestellten Rohprodukte (Rohester) für die Extraktion etwa 20 bis 30 % Fettsäurepolyoxyalkylendiolmonoester und 70 bis 80 % freie Polyoxyalkylendiole sowie geringe Anteile Diester.

Als Extraktionsmittel können lineare, cyclische oder verzweigte Paraffine mit 6 bis 22 C-Atomen, vorzugsweise mit 8 bis 18 C-Atomen, oder auch Gemische davon eingesetzt werden.

Die Extraktion erfolgt bei 40 bis 200 °C, vorzugsweise bei 60 bis 150 °C. Ganz besonders bevorzugt wird bei 90 bis 130 °C extrahiert.

Die Paraffinextraktion kann diskontinuierlich oder vorzugsweise kontinuierlich, beispielsweise in einer Gegenstromextraktionsapparatur, durchgeführt werden.

Nach Abkühlen des Extraktes auf Raumtemperatur scheidet sich aus der Paraffinphase der Fettsäurepolyoxyalkylendiolmonoester in guter Ausbeute und hoher Reinheit ab.

Überraschenderweise gelingt es also, die bei Raumtemperatur in Paraffin nur schlecht löslichen oder unlöslichen Fettsäurepolyoxyalkylendiolmonoester bei erhöhter Temperatur mit Paraffin zu extrahieren und sie dadurch von überschüssigem Polyoxyalkylendiol zu trennen. Anschließend können die Produkte lediglich durch Abkühlung isoliert werden. Es ist also weder eine Destillation des Extraktionsmittels noch ein zweites Lösungsmittel erforderlich.

Das bei der Extraktion abgetrennte überschüssige Polyoxyalkylendiol erfordert keine zusätzlichen Extraktions-oder Reinigungsschritte. Es kann direkt zur Veresterung mit Fettsäuren wieder eingesetzt werden.

Das bei der Abkühlung der Extraktionsphase abgetrennte Paraffin kann für eine weitere Extraktion sofort wieder verwendet werden.

Das vorliegende Extraktionsverfahren ist einfach, effektiv und eignet sich besonders gut für eine kontinuierliche Gegenstromextraktion.

Verfahrensbedingt enthalten die erfindungsgemäß hergestellten Fettsäurepolyoxyalkylendiolmonoester weder Restgehalte an Wasser, anorganischen Salzen oder physiologisch bedenklichen Lösungsmitteln und sind somit insbesondere für Anwendungen im kosmetischen Sektor geeignet.

Die folgenden Beispiele sollen die Erfindung erläutern.

## Beispiel 1

In einen 25 l-Glasrührkolben gibt man 0,86 kg (4 Mol) Kokosfettsäure, 16 kg (20 Mol) Polyoxyethylendiol mit einer mittleren Molmasse von 800 (PEG 800) sowie 10 g p-Toluolsulfonsäure. Bei 140 °C und 10 mbar werden im Verlaufe von 8 Stunden insgesamt 70 g Wasser abdestilliert. Man erhält 16,8 kg Rohester mit einer Säurezahl von 1,1 mg KOH/g und mit einer Esterzahl (EZ) von 28 mg KOH/g. Der Rohester weist 76 % freies Polyoxyethylendiol auf.

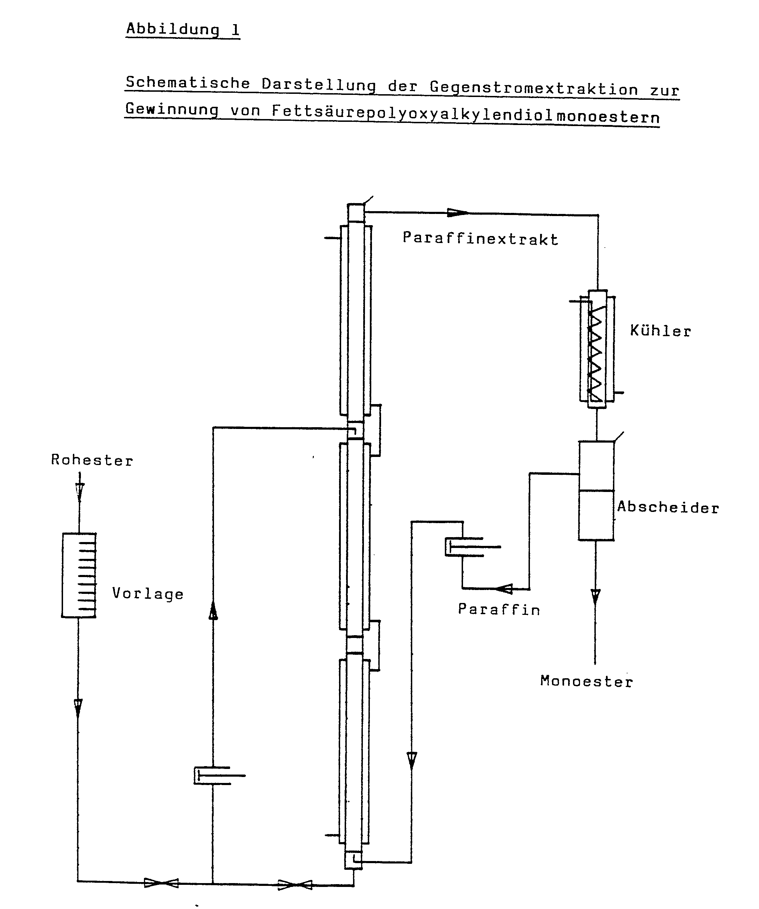

6,3 kg des Rohesters werden in einer kontinuierlichen Gegenstromextraktion (Abbildung 1) bei 100 °C mit 3,4 kg Decan extrahiert.

Im Verlauf von 24 Stunden scheiden sich nach Abkühlen des Paraffinextraktes auf Raumtemperatur insgesamt 1 486 g Produkt folgender Zusammensetzung ab:

92,1 % Kokosfettsäure-PEG-800-monoester
2,5 % Diester
3,4 % Decan
2,0 % PEG 800

Das im Extraktionsrückstand nach Phasentrennung verbliebene PEG 800 wird erneut zur Veresterung eingesetzt.

## Beispiele 2 bis 10

Die Herstellung der Rohester erfolgt analog zu Beispiel 1. Zusammensetzung der Rohester, Extraktionsbedingungen und Ausbeuten an Monoester gehen aus Tabelle 1 hervor.

Tabelle 1:

| Beisp. | Einsatzstoffe und Molverhältnis | Rohester g | EZ mg KOH/g | PEG-Gehalt % | Extraktionsmittel g | T °C | Produkt g | EZ mg KOH/g | Verhältnis Mono-/Diester %[1] | Ausbeute an Monoester %[2] |
|---|---|---|---|---|---|---|---|---|---|---|
| 2 | Kokosfettsäure + PEG 200    1 : 8 | 6 300 | 33 | 78 | Dodecan, 3200 | 120 | 1 228 | 86 | 60 / ? | 53.2 |
| 3 | Kokosfettsäure + PEG 300    1 : 8 | 4 260 | 22 | 81 | Dodecan, 3300 | 120 | 726 | 73 | 62 / ? | 55.7 |
| 4 | Kokosfettsäure + PEG 400    1 : 5 | 5 800 | 26 | 73 | Dodecan, 3300 | 120 | 1 340 | 79 | 82 / 3 | 70.2 |
| 5 | Kokosfettsäure + PEG 600    1 : 5 | 5 930 | 19 | 75 | Decan,    3300 | 90 | 1 441 | 67 | 92 / 3 | 89.5 |
| 6 | Kokosfettsäure + PEG 800    1 : 5 | 6 300 | 15 | 76 | Decan,    3400 | 90 | 1 486 | 53 | 91 / 4 | 89.4 |
| 7 | Kokosfettsäure + PEG 600    1 : 6 | 5 800 | 15 | 79 | C14/17-Paraffin, 3200 | 130 | 1 266 | 69 | 90 / 6 | 93.5 |
| 8 | Kokosfettsäure + PEG 800    1 : 6 | 6 250 | 13 | 80 | C14/17-Paraffin, 3200 | 130 | 1 322 | 54 | 90 / 5 | 95.2 |
| 9 | Laurinsäure + PEG 800    1 : 6 | 6 300 | 14 | 80 | Decan,    3350 | 90 | 1 253 | 54 | 90 / 5 | 89.5 |
| 10 | Palmitinsäure + PEG 800    1 : 6 | 5 950 | 15 | 79 | Dodecan, 3100 | 120 | 1 301 | 53 | 91 / 4 | 94.8 |

[1] Differenz zu 100 %: PEG + Paraffin

[2] bezogen auf theoretisch vorhandenen Monoester im Rohester

## Ansprüche

1. Verfahren zur Herstellung von Fettsäurepolyoxyalkylendiolmonoestern durch Umsetzung von Fettsäuren oder Fettsäurederivaten mit überschüssigen Polyoxyalkylendiolen und anschließende extraktive Reinigung,
dadurch gekennzeichnet,
daß man das Reaktionsgemisch bei erhöhter Temperatur mit Paraffin extrahiert und die Fettsäurepolyoxyalkylendiolmonoester aus dem Paraffinextrakt nach Abkühlung als abgeschiedene Phase gewinnt.

2. Verfahren nach Anspruch 1,
dadurch gekennzeichnet,
daß die Extraktion bei 40 bis 200 °C durchgeführt wird.

3. Verfahren nach Anspruch 2,
dadurch gekennzeichnet,
daß bei 60 bis 150 °C extrahiert wird.

4. Verfahren nach Anspruch 3,
dadurch gekennzeichnet,
daß die Extraktionstemperatur 90 bis 130 °C beträgt.

5. Verfahren nach Anspruch 1,
dadurch gekennzeichnet,
daß die Extraktion mit unverzweigten, verzweigten oder cyclischen Paraffinen mit 6 bis 22 C-Atomen oder mit deren Mischungen durchgeführt wird.

6. Verfahren nach Anspruch 5,
dadurch gekennzeichnet,
daß mit Paraffinen mit 8 bis 18 C-Atomen extrahiert wird.

7. Verfahren nach Anspruch 1,
dadurch gekennzeichnet,
daß man gesättigte und/oder ungesättigte Fettsäuren mit 5 bis 24 C-Atomen einsetzt.

8. Verfahren nach Anspruch 1,
dadurch gekennzeichnet,
daß man Polyoxyethylendiole mit mittleren Molmassen von 150 bis 2 000 verwendet.

9. Verfahren nach Anspruch 8,
dadurch gekennzeichnet,
daß die mittlere Molmasse 400 bis 1 500 beträgt.

10. Verfahren nach den Ansprüchen 1 bis 9,
dadurch gekennzeichnet,
daß man eine kontinuierliche Gegenstromextraktion durchführt und das Paraffin nach Abscheidung der Fettsäurepolyoxyalkylendiolmonoester in die Extraktion zurückführt.

## Abbildung 1

Schematische Darstellung der Gegenstromextraktion zur
Gewinnung von Fettsäurepolyoxyalkylendiolmonoestern

Paraffinextrakt

Kühler

Rohester

Vorlage

Abscheider

Paraffin

Monoester

**Europäisches Patentamt**

**EUROPÄISCHER RECHERCHENBERICHT**

Nummer der Anmeldung

| EINSCHLÄGIGE DOKUMENTE | | | EP 87112973.0 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | SOVIET INVENTIONS ILLUSTRATED, Sektion Ch, Woche B25, 1. August 1979<br><br>DERWENT PUBLICATIONS LTD., London, D13<br><br>   * SU-620 474 (MOSC FATS RES INST) *<br><br>-- | 1-3,5 | C 07 C 69/28<br>C 07 C 67/58 |
| A | DD - A - 87 315 (W.GERHARDT et al.)<br><br>   * Beispiele; Ansprüche *<br><br>-- | 1,7-9 | |
| D,A | DE - C - 1 175 220 (HOECHST)<br><br>   * Beispiel; Ansprüche *<br><br>---- | 1,7-9 | |
| | | | **RECHERCHIERTE SACHGEBIETE (Int. Cl.4)**<br><br>C 07 C 67/00<br>C 07 C 69/00 |

Der vorliegende Recherchenbericht wurde für alle Patentansprüche erstellt.

| Recherchenort | Abschlußdatum der Recherche | Prüfer |
|---|---|---|
| WIEN | 13-01-1988 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82